# EUROPEAN PATENT APPLICATION

(11) **EP 4 149 210 A1**
(43) Date of publication of application: **15.03.2023**
(21) Application number: 21196127.1
(22) Date of filing: 10.09.2021
(51) Int. Cl.: H05B 45/20, H05B 47/175, H05B 47/16, A61N 5/06, A61M 21/00

(54) **CONTROLLING A LIGHT ENVIRONMENT AT DIFFERENT LOCATIONS IN A TRANSPORT SHUTTLE**

(71) Applicant: BrainLit AB, 223 63 Lund (SE)
(72) Inventor: Wingren, Tord, 234 39 Lomma (SE); Singvall, Jacob, 237 32 Bjärred (SE); Lindoff, Bengt, 237 35 Bjärred (SE)
(74) Representative: AWA Sweden AB

(57) **Abstract**

A lighting system control engine for controlling a light environment at different locations in a transport shuttle is disclosed. The lighting system control engine comprising circuitry configured to execute: a user association function configured to associate an individual user with each of the different locations; a light profile setting function configured to, for each of the different locations, form a location specific light profile by manipulating a target light profile describing illuminance and spectrum over a time period by inducing a time shift on the target light profile, wherein a phase for the induced time shift is dependent on: a time zone of an original place of departure of the individual user associated with the location, and a time zone of a target arrival destination of the individual user associated with the location; and a light source control function configured to individually control a light environment at each of the different locations in accordance with the respective location specific light profiles. Also, a lighting system including the control engine and a method for controlling light environments at the different locations are disclosed.

## Description

### Technical field

The present disclosure relates to control of light environments at different locations in a transport shuttle, such as an airplane, a high-speed train, a long-haul bus or a hyperloop vessel.

### Background of the invention

Chronobiology, which is a field of biology that studies timing (e.g., periodic) processes and phenomena in living organisms, is becoming increasingly more important for understanding human physiology. For example, the circadian rhythm is an internal process of living organisms (e.g. humans) that regulates the sleep-wake cycle. The circadian rhythm typically repeats over a time period which, for most people, is slightly longer than 24 hours. It is also known that this cycle can be different between different individuals. For example, some persons are known to be "morning" persons, while other are "evening" persons. Hence, the behavior at a certain time of day for different individual typically varies. This is usually referred to as a person's chronotype.

An important part of chronobiology is the study of the impact of light on living organisms. It has, for example, been found that exposing a person to bright light in the evening can postpone the time that person falls asleep. Recent studies have further found that light can affect a person's alertness and performance. However, the effect of light at a certain time of the day is not only individual and dependent on time of the day, it also depends on the individual persons light exposure during the day. For instance, in case the person has been exposed to day light in the morning (e.g., light similar to that of a bright summer's morning), the effect of bright light in the afternoon is typically less than is the person had spent the morning being exposed to light of lower illuminance (e.g. artificial light inside an office).

In case of travelling with airplanes especially long-haul flights causing jet-lag, the circadian rhythm is disturbed. Such disturbance, especially if it happens frequently, induce a higher risk of medical conditions such as cancer and cardiovascular disease, as well as depression. The light environment onboard airplanes of today does not take the human circadian rhythm into much account and therefore does not optimize the wellbeing for the individual persons travelling with the airplane. Therefore, there is a need in improving the light environment onboard airplanes.

### Summary of the invention

It is an object to, at least partly, mitigate, alleviate, or eliminate one or more of the above-identified deficiencies in the art and disadvantages singly or in any combination and solve at least the above-mentioned problem.

According to a first aspect a light system control engine for controlling a light environment at different locations in a transport shuttle is provided: The lighting system control engine comprising circuitry configured to execute: a user association function configured to associate an individual user with each of the different locations; a light profile setting function configured to, for each of the different locations, form a location specific light profile by manipulating a target light profile describing illuminance and spectrum over a time period by inducing a time shift on the target light profile, wherein a phase for the induced time shift is dependent on: a time zone of an original place of departure of the individual user associated with the location, and a time zone of a target arrival destination of the individual user associated with the location; and a light source control function configured to individually control a light environment at each of the different locations in accordance with the respective location specific light profiles. By applying the individual light profiles for the different passengers, formed according to the above, optimization of the light environment for the individual passengers is provided for. Especially, the light environment for the individual passengers can be set such that a passenger's adoption to the time zone of the target arrival destination can be accelerated. This will provide for a reduction of the effect from disturbance circadian rhythm due to travelling across time-zones.

The target light profile may be a common target light profile for the individual users.

The circuitry may further be configured to execute a target light profile retrieving function configured to retrieve an individual target light profile for one or more individual users by accessing a database comprising individual target light profiles associated with individual users. The light profile setting function may be configured to form location specific light profiles for the individual users having an individual target light profile associated thereto. This by manipulating the retrieved individual target light profile by inducing a time shift on the retrieved target light profile. Taking into account an individual target light profile the chronotype of the individual may be accounted for. Further, other personal criteria such as medical status, sleep pattern etc. may be accounted for.

Each of the different locations in the transport shuttle may be associated with a passenger seat. The user association function may be configured to associate an individual user with a location by accessing a database comprising a passenger manifest. The passenger manifest preferably comprising information pertaining to an original place of departure and a target arrival destination. Especially, a time zone of the original place of departure and a time zone of the target arrival destination. The passenger manifest also comprising information pertaining to an allotted seat/location for the passenger in the transport shuttle.

The target light profile may be describing illuminance and spectrum for a day and night cycle.

The phase for the induced time shift may further be dependent on an individual jet-lag capability factor of the individual user associated with the location.

According to a second aspect a lighting system in a transport shuttle is provided. The lighting system comprising a plurality of light sources each being associate with a different location within the transport shuttle; and a light system control engine according to the first aspect. The light system control engine is configured to control the plurality of light sources.

The lighting system may further comprise a plurality of check-in access points each being associated with one or more of the different locations. The check-in access points may be being configured to allow a user to check-in at a specific location. The user association function may be configured to associate an individual user with each of the different locations in accordance with the check-in.

The check-in access points may be configured to communicate with a smart device associated with a user in order to check-in a specific user at the specific location.

The above-mentioned features of the light system control engine, when applicable, apply to this second aspect as well. In order to avoid undue repetition, reference is made to the above.

According to a third aspect a transport shuttle comprising a light system control engine according to the second aspect or a lighting system according to the second aspect is provided. The above-mentioned features of the light system control engine or the lighting system, when applicable, apply to this third aspect as well. In order to avoid undue repetition, reference is made to the above.

According to a fourth aspect a method for controlling light environments at different locations in a transport shuttle is provided. The method comprising the following steps. For each of the different locations, associating an individual user with the location. For each of the different locations, forming a location specific light profile by manipulating a target light profile describing illuminance and spectrum over a time period by inducing a time shift on the target light profile, wherein a phase for the induced time shift is dependent on a time zone of an original place of departure of the individual user associated with the location, and a time zone of a target arrival destination of the individual user associated with the location. Individually controlling a light environment at each of the different locations in accordance with the respective location specific light profiles.

The method may further comprise retrieving an individual target light profile for one or more of the individual users by accessing a database comprising individual target light profiles associated with individual users. Forming a location specific light profile for a location associated with a user having an individual target light profile associated to the user may comprise manipulating the retrieved individual target light profile by inducing a time shift on the retrieved individual target light profile.

Each of the different locations in the transport shuttle may be associated with a passenger seat. Associating an individual user with a location may comprise associating an individual user with a seat by accessing a database comprising a passenger manifest.

The transport shuttle may comprise one or more check-in access point associated with the different locations. Associating an individual user with a location may comprise checking-in at a check-in access point.

The above-mentioned features of the light system control engine or the lighting system, when applicable, apply to this fourth aspect as well. In order to avoid undue repetition, reference is made to the above.

According to a fifth aspect a non-transitory computer-readable storage medium is provided. The storage medium having stored thereon instructions for implementing the method according to the fourth aspect, when executed on a device having processing capabilities. The above-mentioned features of the light system control engine or the lighting system, when applicable, apply to this fifth aspect as well. In order to avoid undue repetition, reference is made to the above.

A further scope of applicability will become apparent from the detailed description given below. However, it should be understood that the detailed description and specific examples are given by way of illustration only.

It is to be understood that the terminology used herein is for purpose of describing particular embodiments only, and is not intended to be limiting. It must be noted that, as used in the specification and the appended claim, the articles "a," "an," "the," and "said" are intended to mean that there are one or more of the elements unless the context clearly dictates otherwise. Thus, for example, reference to "a unit" or "the unit" may include several devices, and the like. Furthermore, the words "comprising", "including", "containing" and similar wordings does not exclude other elements or steps.

### Brief description of the drawings

The above and other aspects will now be described in more detail, with reference to appended figures. The figures should not be considered limiting; instead they are used for explaining and understanding.

As illustrated in the figures, the sizes of layers and regions may be exaggerated for illustrative purposes and, thus, are provided to illustrate the general structures. Like reference numerals refer to like elements throughout.
Fig. 1 illustrates a lighting system arranged in a transport shuttle.
Fig. 2 illustrates a control engine of the lighting system of Fig. 1.
Fig. 3 illustrates manipulation of a target light profile for forming location specific light profiles.
Fig. 4 is a block diagram of a method for controlling a light environment at different locations in a transport shuttle.

### Detailed description

The present invention will now be described more fully hereinafter with reference to the accompanying drawings, in which currently preferred embodiments of the invention are shown. This invention may, however, be embodied in many different forms and should not be construed as limited to the embodiments set forth herein; rather, these embodiments are provided for thoroughness and completeness, and to fully convey the scope of the invention to the skilled person.

In case of travelling with transport shuttles having departure and arrival destinations at different time zones the circadian rhythm is disturbed. Such disturbance, especially if it happens frequently, induce a higher risk of medical conditions such as cancer and cardiovascular disease, as well as depression. The transport shuttle can for instance be an airplane, a high-speed train, a long-haul bus or a hyperloop vessel configured to perform long-haul travelling crossing a plurality of time-zones. Passengers on-board the transport shuttle typically have different original place of departure and/or different target arrival destination. In this context an original place of departure is a place where a passenger has started his or her travel and a target arrival destination is a destination at which the passengers travel ends or at least stops for a period of time. Hence, the target arrival destination may be a final arrival destination (i.e. the final goal for this particular travel) for the passenger or the target arrival destination may be destination at which the passenger is stopping for e.g. transferring to another transport shuttle. Accordingly, the travel with a current transport shuttle may be just a part of the travel conducted be a specific passenger. Accordingly, the passengers on the transport shuttle may have started the traveling at different original place of departure and/or ending the traveling at different target arrival destination. Accordingly, the circadian rhythm for the passengers on-board the transport shuttle may be in different phases. Further, a time-zone difference for the different passengers may also differ.

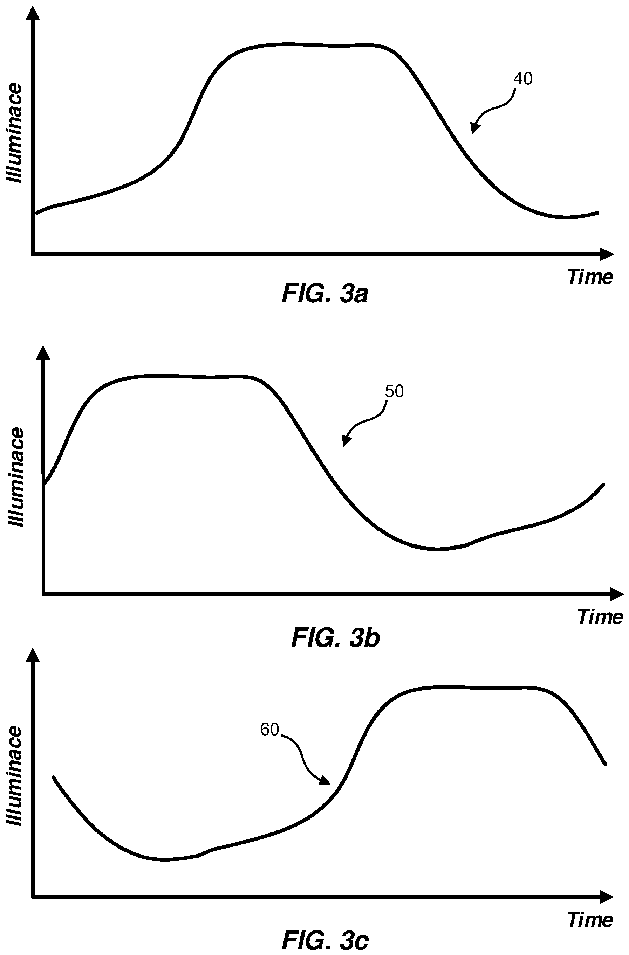

The concept of the present invention is independently controlling light sources at a plurality of locations in the transport shuttle. Each of the different locations may each be associated with a passenger seat in the transport shuttle. The light sources are controlled such that a light environment is set to be different at the different locations. Within the context of this disclosure, the wording "light environment" should be construed as spectrally and temporally resolved amount of light emitted from light source(s) at the location. The light source(s) is/are controlled using a light profile associated with the respective location. Such a location specific light profile comprises spectrally and temporally resolved information about amount of light to be emitted from light source(s) at the specific location. Since the information is spectrally and temporally resolved, the location specific light profile comprise information on the amount of light at which time and at which wavelength/color the light source(s) at a specific location is/are set to emit. The light profile at different locations in the transport shuttle is set to be time-shifted as compared to a target light profile. The time-shift is to be made taking into account the individual/passenger associated with the specific location. The phase for the time shift may be dependent on a time zone of an original place of departure of the passenger associated with the specific location. Alternatively, or in combination, the phase for the time shift may be dependent on a time zone of a target arrival destination of the passenger associated with the specific location. Alternatively, or in combination, the phase for the time shift may be dependent on an individual jet-lag capability factor of the passenger associated with the specific location. The jet-lag capability factor being a factor on how much an individual target light profile can be compressed as compared with a typical day and night cycle. An individual jet-lag capability factor for an individual can be determined by asking the individual questions beforehand. Such questions may e.g. be how he/she feel from jet-lag and how long time he/she has taken to recover from jet-lag. The questions may have been asked via an application run on a smart device associated with the individual. Input given by the individual may be stored in a user profile associated with the individual. Based on input an individual target profile associated with the individual can be determined. The faster recovery, the more compressed an individual target profile can be as compared with a typical day and night cycle and vice versa. Instead of asking, similar information may be obtained from a monitoring device associated with the individual. The smart device may e.g. monitor heart rate, blood pressure, body temperature, stress hormone levels etc. at historical travels including jet-lag issues. Such data monitored during historical travels may then be compared to data monitored during non-jet-lag days. By such monitoring, information pertaining to how an individual is reacting to travels between different time-zones can be obtained. This information may then be used to determine a jet-lag capability factor for the individual. How to form the light profiles for the different location will be discussed in more detail below, especially in connection with Fig. 3.

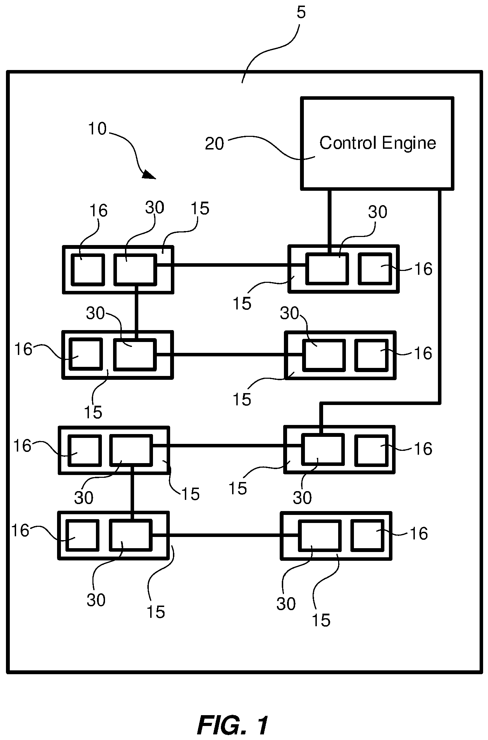

Fig. 1 illustrates a lighting system 10 arranged in a transport shuttle 5, e.g. an airplane, a high-speed train, a long-haul bus or a hyperloop vessel. The transport shuttle 5 comprises a plurality of locations 15. Each location 15 is preferably associated with a separate passenger seat of the transport shuttle 5. The lighting system 10 comprises a plurality of light sources 30. A subset of the plurality of light sources 30 is located at each location 15. Each subset of the plurality of light sources 30 may be one or more light sources 30. In case a subset of the plurality of light sources 30 is two or more light sources 30, the light sources 30 may be of a same or different type. The light sources 30 of the same or different type may be controlled individually or jointly. The lighting system 10 further comprises a control engine 20. The control engine 20 is configured to control illumination of the plurality of light sources 30. More specifically, the control engine 30 is configured to control illumination of each of the subsets of light sources 30 individually. Hence, the control engine 30 is configured to control illumination in each location 15 individually. As will be discussed in greater detail below, the control engine 30 is configured to base the controlling at the different locations 15 on a light profile dedicated for the specific location 15.

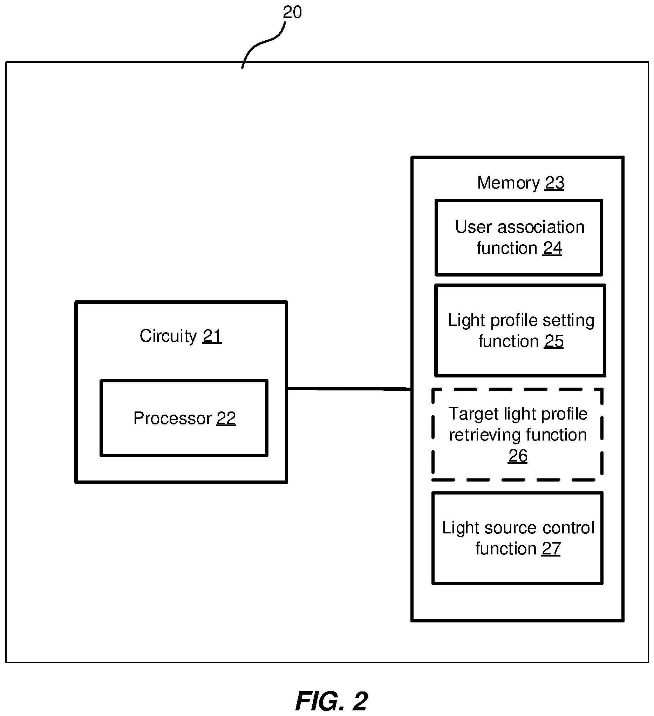

In connection with Fig. 2, the control engine 20 will be discussed in more detail. The control engine 20 may be embodied in a specific device, e.g. a server. However, as readily understood by the skilled person the control engine 20 may be distributed over a plurality of devices. The control engine 20 comprises circuitry 21. The circuitry 21 may include a processor 22, such as a central processing unit (CPU), microcontroller, or microprocessor. The circuitry 21 is configured to execute program code stored in a memory 23, in order to carry out functions and operations of the control engine 20.

The memory 23 may be one or more of a buffer, a flash memory, a hard drive, a removable medium, a volatile memory, a non-volatile memory, a random access memory (RAM), or another suitable device. In a typical arrangement, the memory 23 may include a non-volatile memory for long term data storage and a volatile memory that functions as system memory for the control engine 20. The memory 23 may exchange data with the circuitry 21 over a data bus. Accompanying control lines and an address bus between the memory 23 and the circuitry 21 also may be present.

Functions and operations of the control engine 20 may be embodied in the form of executable logic routines (e.g., lines of code, software programs, etc.) that are stored on a non-transitory computer readable medium (e.g., the memory 23) of the control engine 20 and are executed by the processor 21. Furthermore, the functions and operations of the control engine 20 may be a stand-alone software application or form a part of a software application that carries out additional tasks related to the control engine 20. The described functions and operations may be considered as part of a method that the control engine 20 is configured to carry out. Also, while the described functions and operations may be implemented in software, such functionality may as well be carried out via dedicated hardware or firmware, or some combination of hardware, firmware and/or software.

The control engine 20 is configured to execute a user association function 24. The user association function 24 is configured to associate an individual user with each of the different locations 15. Such association may be made by accessing a database comprising a passenger manifest. The passenger manifest comprising information on which passenger is being allocated a specific seat (i.e. location 15) in the transport shuttle 5. Alternatively, or in combination, the transport shuttle 5 may be equipped with check-in access points 16. The check-in access points 16 being configured to allow a user to check-in at a specific location 15. The user association function may hence be configured to associate an individual user/passenger with a specific location 15 in accordance with the check-in. Each location may comprise a specific check-in access point 16. Alternatively, a common check-in access point 16 may service a plurality of locations 15 (i.e. seats in the transport shuttle 5). A check-in access point 16 may be configured to communicate with a smart device, such as a smartphone, smart watch, or an RF ID tag associated with a passenger in order to check-in the passenger at the specific location 15. Alternatively, or in combination, a check-in access point 16 may be configured to identify a passenger using biometric data, such as face recognition, fingerprint recognition or the like. It is readily understood that other ways of associating a specific passenger to a specific location may also be used.

The control engine 20 is further configured to execute a light profile setting function 25. The light profile setting function 25 is configured to form a location specific light profile for each of the different locations 15. The location specific light profile is to be formed taking into account which specific passenger being associated with the specific location 15. The light profile setting function 25 is set to form the different location specific light profiles by manipulating a target light profile describing illuminance and spectrum over a time period. Typically, the target light profile is describing illuminance and spectrum for a day and night cycle. The target light profile may be a common target light profile for a plurality of the individual users/passengers. Alternatively, or in combination, one or more of the different passengers may have an individual target light profile associated to him/her. Such an individual target light profile may take into account information about a chronotype for the passenger, a historic light dose received by the passenger and/or a medical condition of the passenger. Individual target light profiles associated to individuals may be retrieved by the control engine 20 executing a target light profile retrieving function 26. The target light profile retrieving function 26 is configured to retrieve an individual target light profile for passengers having such individual target light profile associated to him/her. The target light profile retrieving function 26 may be set to access a database comprising individual target light profiles associated with individual users. This in order to retrieve an individual target light profiles for the passengers of the transport shuttle 5.

The light profile setting function 25 is configured to, for each location 15, manipulate the target light profile associated with the user associated to the location 15. The manipulation comprises inducing a time shift on the target light profile associated with the user associated to the location 15. The phase for the induced time shift is dependent on a time zone of the original place of departure of the individual user associated with the location and on the time zone of a target arrival destination of the individual user associated with the location. Alternatively, or in combination, the phase for the induced time shift is dependent on the individual jet-lag capability factor of the individual user associated with the location. Hence, light profile setting function 25 is configured to form individual light profiles for the different passengers. By applying the individual light profiles for the different passengers, the light environment for the individual passengers will be optimized so that adoption to the time zone of the target arrival destination can be accelerated. This will reduce the effect of jet-lag.

Examples of manipulated light profiles 40, 50, 60 are illustrated in Fig. 3. The light profiles 40, 50, 60 are describing illuminance and spectrum over a time period. Typically, the light profiles 40, 50, 60 are describing illuminance and spectrum for a day and night cycle (24 hours). In each of the in Fig. 3 illustrated light profiles 40, 50, 60 just a part of the template light profile 40, 50, 60 for one specific wavelength of light is illustrated. The illuminance for different wavelengths of the light spectrum will vary in a similar manner over time. However, as readily understood by the skilled person e.g. red and blue light may vary differently throughout a day and night cycle. The in Fig. 3a illustrated light profile 40 is a light profile with no phase shift induced thereto. Hence, the light profile 40 mimics the target light profile without any phase shift. The in Fig. 3b illustrated light profile 50 is a light profile with a phase shift towards earlier time, as compared with a target light profile, induced thereto. The in Fig. 3c illustrated light profile 60 is a light profile with a phase shift towards later time, as compared with a target light profile, induced thereto.

The control engine 20 is further configured to execute a light source control function 27. The light source control function 27 is configured to individually control a light environment at each of the different locations in accordance with the respective location specific light profiles. In order to achieve this the light source control function 27 is configured to control the one or more light sources 30 located at each location 15. Hence, the one or more light sources 30 located at each location 15 is independently controlled so as to achieve an individual light environment at each location 15 in the transport shuttle 5.

Hence, the control engine 20 is configured to individually control the light sources 39 at the different locations15 in the transport shuttle 5. The light profile, and hence the light environment, at the different locations 15 is set to be manipulated based on the individual passengers on-board the transport shuttle 5. Typically, the light profiles are time shifted between different locations 15. This could for instance be the case in an inter-continental flight for an airplane, where a light source for each seat (location) has different time shifted light profiles, depending on the respective passenger's individual circadian phase. The information about respective passenger's individual circadian phase may be comprised in a passenger manifest comprising information about original departure place and target arrival destination. Hence, the light environment at the respective location/seat can be adjusted to correct "time of the day" for respective passenger. The target light profile for a day and night cycle may be common for all individual passengers/users. Alternatively, some or all the passengers/users may have individual target light profiles assigned to him or her.

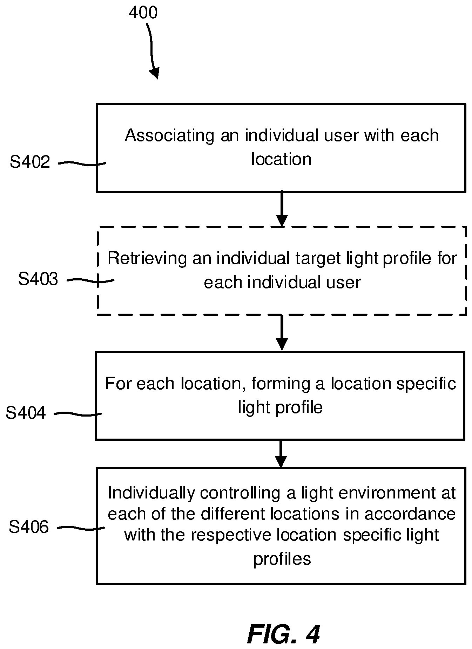

In connection with Fig. 4 a method 400 for controlling light environments at different locations 15 in a transport shuttle 5 will be discussed. Some of all the steps of the method 400 may be performed by the functions of the control engine 20 described above. However, it is equally realized that some or all of the steps of the method 400 may be performed by similar functions performed at other devices. The method 400 comprises the following steps. The steps may be performed in any suitable order. The method comprises the following steps.

For each of the different locations, associating S402 an individual user with the location. Associating S402 an individual user with a location may comprise associating an individual user with a seat in the transport shuttle 5 by accessing a database comprising a passenger manifest. Associating S402 an individual user with a location may comprise checking-in at a check-in access point 16 as discussed above.

For each of the different locations, forming S404 a location specific light profile. This by manipulating a target light profile describing illuminance and spectrum over a time period by inducing a time shift on the target light profile. The target light profile may be a common target light profile for the individual users. However, one or more of the individual users may be associated with an individual target light profile. If so, such an individual target light profile may be retrieved by retrieving S403 an individual target light profile for the individual user by accessing a database comprising individual target light profiles associated with individual users. Further, in this case forming S404 a location specific light profile for the location associated with the user having an individual target light profile associated to the user comprises manipulating the retrieved individual target light profile of the individual user by inducing an time shift on the retrieved individual target light profile. Common for all manipulations is that a phase for the induced time shift is dependent on a time zone of an original place of departure of the individual user associated with the location, and a time zone of a target arrival destination of the individual user associated with the location.

Individually controlling S406 a light environment at each of the different locations in accordance with the respective location specific light profiles.

The person skilled in the art realizes that the present invention by no means is limited to what is explicitly described above. On the contrary, many modifications and variations are possible within the scope of the appended claims.

For example, upon planning where to seat the different passengers, information about the passenger's original departure place and target arrival destination can be taken into account. For example, passengers being at similar "time of the day" can be clustered together so that their respective light environments does not affect each other to much.

Moreover, a duration of a stay for a transfer to another transport shuttle may be considered upon manipulating the target light profile when forming a location specific light profile.

Furthermore, a time duration of the stay at the target destination, or other further travels nearby in time (after a current travel) may be taken into account upon manipulating the target light profile when forming a location specific light profile.

Additionally, variations can be understood and effected by the skilled person in practicing the claimed invention, from a study of the drawings, the disclosure, and the appended claims.

## Claims

1. A light system control engine for controlling a light environment at different locations (15) in a transport shuttle (5), the lighting system control engine comprising circuitry (21) configured to execute:
a user association function (24) configured to associate an individual user with each of the different locations (15);
a light profile setting function (25) configured to, for each of the different locations (15), form a location specific light profile (40, 50, 60) by manipulating a target light profile describing illuminance and spectrum over a time period by inducing a time shift on the target light profile, wherein a phase for the induced time shift is dependent on: a time zone of an original place of departure of the individual user associated with the location (15), and a time zone of a target arrival destination of the individual user associated with the location (15); and
a light source control function (27) configured to individually control a light environment at each of the different locations (15) in accordance with the respective location specific light profiles (40, 50, 50).

2. The light system control engine according to claim 1, wherein the target light profile is a common target light profile for the individual users.

3. The light system control engine according to claim 1, wherein the circuitry (21) is further configured to execute a target light profile retrieving function (26) configured to retrieve an individual target light profile for one or more individual users by accessing a database comprising individual target light profiles associated with individual users, wherein the light profile setting function is configured to form location specific light profiles for the individual users having an individual target light profile associated thereto by manipulating the retrieved individual target light profile of the respective individual user by inducing a time shift on the retrieved individual target light profile.

4. The light system control engine according to any one of claims 1-3, wherein each of the different locations (15) in the transport shuttle (5) is associated with a passenger seat, wherein the user association function (24) is configured to associate an individual user with a location (15) by accessing a database comprising a passenger manifest.

5. The light system control engine according to any one of claims 1-4, wherein the target light profile is describing illuminance and spectrum for a day and night cycle.

6. The light system control engine according to any one of claims 1-5, wherein the phase for the induced time shift is further dependent on an individual jet-lag capability factor of the individual user associated with the location.

7. A lighting system in a transport shuttle (5), the lighting system comprising:
a plurality of light sources (30) each being associate with a different location (15) within the transport shuttle (5); and
a light system control engine (20) according to any one of claims 1-6, wherein the light system control engine (20) is configured to control the plurality of light sources (30).

8. The lighting system according to claim 7, further comprising a plurality of check-in access points (16) each being associated with one or more of the different locations (15), the check-in access points (16) being configured to allow a user to check-in at a specific location (15),
wherein the user association function is configured to associate an individual user with each of the different locations (15) in accordance with the check-in.

9. The lighting system according to claim 8, wherein the check-in access points (16) are configured to communicate with a smart device associated with a user in order to check-in a specific user at the specific location (15).

10. A method for controlling light environments at different locations in a transport shuttle, the method comprising:
for each of the different locations, associating (S402) an individual user with the location;
for each of the different locations, forming (S404) a location specific light profile by manipulating a target light profile describing illuminance and spectrum over a time period by inducing a time shift on the target light profile, wherein a phase for the induced time shift is dependent on a time zone of an original place of departure of the individual user associated with the location, and a time zone of a target arrival destination of the individual user associated with the location; and
individually controlling (S406) a light environment at each of the different locations in accordance with the respective location specific light profiles.

11. The method according to claim 10, wherein the target light profile is a common target light profile for the individual users.

12. The method according to claim 10, further comprising:
retrieving (S403) an individual target light profile for one or more of the individual users by accessing a database comprising individual target light profiles associated with individual users,
wherein forming (S404) a location specific light profile for a location associated with a user having an individual target light profile associated to the user comprises manipulating the retrieved individual target light profile by inducing a time shift on the retrieved individual target light profile.

13. The method according to any one of clams 10-12, wherein the different locations in the transport shuttle are each associated with a passenger seat, wherein associating (S402) an individual user with a location comprises associating an individual user with a seat by accessing a database comprising a passenger manifest.

14. The method according to any one of clams 10-12, wherein the transport shuttle comprises one or more check-in access point associated with the different locations, wherein associating (S402) an individual user with a location comprises checking-in at a check-in access point.

15. A non-transitory computer-readable storage medium having stored thereon instructions for implementing the method according to any one of claims 10-14, when executed on a device having processing capabilities.
